# EUROPEAN PATENT SPECIFICATION

(11) **EP 2 775 852 B1**
(45) Date of publication and mention of the grant of the patent: **17.08.2016**
(21) Application number: 12772784.0
(22) Date of filing: 12.10.2012
(51) Int. Cl.: A23L 29/206, A23L 33/10

(54) **COMPOSITION FOR USE IN BRAIN GROWTH AND/OR COGNITIVE AND/OR PSYCHOMOTOR DEVELOPMENT**
ZUSAMMENSETZUNG ZUR VERWENDUNG MIT GEHIRNWACHSTUM UND/ODER KOGNITIVER UND/ODER PSYCHOMOTORISCHER ENTWICKLUNG
COMPOSITION À UTILISER DANS LE DÉVELOPPEMENT PSYCHOMOTEUR ET/OU COGNITIVE ET/OU CROISSANCE CÉRÉBRALE

(30) Priority: 18.10.2011 EP 11185600
(43) Date of publication of application: 17.09.2014
(60) Divisional application: 16174616.9
(73) Proprietor: Nestec S.A., 1800 Vevey (CH)
(72) Inventor: GARCIA-RODENAS, Clara, CH-1072 Forel (CH); ORNSTEIN, Kurt, CH-1073 Savigny (CH)
(74) Representative: Corticchiato, Olivier
(86) International application number: PCT/EP2012/070281
(87) International publication number: WO 2013/057049

(56) References cited:
- EP-A1- 2 258 216
- EP-A2- 2 272 381
- WO-A1-2007/101675
- US-A1- 2008 003 330

## Description

### Field of the invention

This invention relates to a composition for use in brain growth and/or cognitive and/or psychomotor development. This composition is for use in mammals, preferably in humans, more preferably in infants.

### Background of the invention

During development, especially the first few years of life, children show interesting patterns of neural development and a high degree of neuroplasticity. The relation of brain development and cognitive development is extremely complex and, since the 1990s, a growing area of research.

Some new development intends to prove a link between brain growth and cognitive development in infants and particularly in preterm infants or extremely low gestational age newborns (ELGANs) *(*J. Pediatr. 2009; 155:344-9*).*

Thus there is a great interest in promoting brain growth, particularly in preterm infants, so that to favor their cognitive and/or psychomotor development.

Supplementation with long-chain poly-unsaturated fatty acids has been proposed to promote cognitive and psychomotor development and number clinical trials have investigated this hypothesis. However, a recent meta-analysis that combined these data showed no significant effect of supplementation on neurodevelopment (Schulzke SM, Patole SK, Simmer K, Longchain polyunsaturated fatty acid supplementation in preterm infants (Review) The Cochrane Library 2011, Issue 2).

Cognitive development can be also improved in preterm infants by largely increasing the protein and energy intake (I Brandt, E.J. Sticker and M.J. Lentze, catch-up growth of head circumference of very low birth weight, small for gestational age preterm infants and mental development to adulthood, J Pediatr 2003;142:463-8). However, large enteral feeding volumes and/or high protein/energy density of the feeds can induce intolerance to feeding. In addition, high protein intake, which leads to increased urea production, may increase the risk of renal insufficiency and metabolic acidosis in preterm infants. Furthermore, high protein/energy intake during infancy has been associated to long-term alterations on metabolic health (increased risk of obesity, type II diabetes and cardiovascular disease) (KK ONG & RJF LOOS, Rapid infancy weight gain and subsequent obesity: Systematic reviews and hopeful suggestions Acta Paediatrica, 2006; 95: 904┐ 908; J Rotteveel, MM van Weissenbruch, JWR Twisk, HA Delemarre-Van de Waal, Infant and Childhood Growth Patterns, Insulin Sensitivity, and Blood Pressure in Prematurely Born Young Adults Pediatrics 2008;122:313-321).

There is a need for a nutritional composition for use in brain growth and/or cognitive and/or psychomotor development, in particular in infants and young children, preferably infants, who were born preterm or with low-birth weight (LBW) or experienced intra-uterine growth retardation (IUGR) or who suffered from growth stunting because of malnutrition, such as suboptimal intra-uterine nutrition, and/or disease.

There is more generally a need for this nutritional intervention in young mammals, in particular infants and children, preferably infants, but also young pets.

### Summary of the invention

The present inventors have found surprisingly that the administration of a mixture of specific oligosaccharides in combination with at least one long chain polyunsaturated fatty acid (LC-PUFA) and at least one probiotic, is particularly effective in brain growth and/or cognitive and/or psychomotor development.

Accordingly, the present invention provides a composition comprising at least one LC-PUFA, at least one probiotic and a mixture of oligosaccharides, said mixture containing at least one N-acetylated oligosaccharide, at least one sialylated oligosaccharide and at least one neutral oligosaccharide, for use in brain growth and/or cognitive and/or psychomotor development.

The composition according to the invention is preferably a nutritional composition.

The LC-PUFA is preferably chosen among arachidonic acid (ARA) and docosahexanoic acid (DHA), more preferably the LC-PUFA is a mixture of ARA and DHA.
The probiotic is preferably chosen among probiotic bacterial strains, more preferably the probiotic is a *lactobacillus* or a *bifidobacterium.* In a preferred embodiment, the probiotic is *Bifidobacterium lactis* and *Lactobacillus reuteri.*

The neutral oligosaccharide is preferably chosen among fructooligosaccharides (FOS) and galactooligosaccharides (GOS), preferably GOS.

In one embodiment the oligosaccharide mixture may be derived from animal milk, such as one or more of cow, goat, sheep or buffalo milk. For example, it was obtained by cow's milk fractionation and further enzymatic treatment.

In a second embodiment the oligosaccharide mixture may be prepared using enzymatic, chemo-enzymatic and/or chemical means.

In a third embodiment the oligosaccharide mixture may be prepared using yeast and/or bacterial fermentation technologies. For example, yeast and/or bacterial cells expressing suitable enzymes such as glycosidases and/or glycosyltransferases upon genetic modification or not might be used to this end.

The composition of the invention is preferably used for infants who were born preterm or with low-birth weight (LBW) or experienced intra-uterine growth retardation (IUGR) and/or who suffered from growth delays because of disease and/or malnutrition.

### Detailed description of the invention

As used herein, the following terms have the following meanings.

The term "child" means a human between the stages of birth and puberty. An adult is a human older than a child.

The term "infant" means a child under the age of 12 months.

The term "preterm infant" (or "premature infant") means an infant born at least than 37 weeks gestational age.

The term "low birth weight infant" means an infant having a liveborn weight less than 2,500 g.

The term "young child" means a child aged between one and three years.

The term "infant formula" means a foodstuff intended for particular nutritional use by infants during the first four to six months of life and satisfying by itself the nutritional requirements of this category of person (Article 1.2 of the European Commission Directive 91/321/EEC of May 14, 1991 on infant formulae and follow-on formulae).

The term "preterm infant formula" means an infant formula intended for a preterm infant.

The term "human milk fortifier" means a supplement used to increase the calories, protein, minerals and vitamins in breast milk fed to preterm infants or infants with a low birth weight.

The term "follow-on formula" means a foodstuff intended for particular nutritional use by infants aged over four months and constituting the principal liquid element in the progressively diversified diet of this category of person.

The term "starter infant formula" means a foodstuff intended for particular nutritional use by infants during the first four months of life.

The term "baby food" means a foodstuff intended for particular nutritional use by infants during the first years of life.

The term "infant cereal composition" means a foodstuff intended for particular nutritional use by infants during the first years of life.

The term "growing-up milk" means a milk-based beverage adapted for the specific nutritional needs of young children.

The term "weaning period" means the period during which the mother's milk or the infant formula is partially or totally substituted by other food in the diet of an infant.
The term "brain growth and/or cognitive and/or psychomotor development" means the support of brain growth and/or of cognitive and/or of psychomotor development.

The term "nutritional composition" means a composition which nourishes a subject. This nutritional composition is usually to be taken orally, intragastrically, or intravenously, and it usually includes a lipid or fat source and a protein source.

The term "synthetic mixture" means a mixture obtained by chemical and/or biological means, which can be chemically identical to the mixture naturally occurring in mammalian milks.

The term "hypoallergenic composition" means a composition which is unlikely to cause allergic reactions.

The term "probiotic" means microbial cell preparations or components of microbial cells or microbial cell metabolites with a beneficial effect on the health or well-being of the host. (Salminen S, Ouwehand A. Benno Y. et al. "Probiotics: how should they be defined" Trends Food Sci. Technol. 1999:10 107-10).

The term "oligosaccharide" means a carbohydrate having a degree of polymerisation (DP) ranging from 2 to 20 inclusive but not including lactose.

The term "neutral oligosaccharide" means an oligosaccharide having no charge and no N-acetyl residue.

The term "sialylated oligosaccharide" means an oligosaccharide having a sialic acid (such as N-acetylneuraminic acid and/or N-glycolylneuraminic acid) residue.

The term "N-acetylated" oligosaccharide means an oligosaccharide having at least one hexose carrying an N-acetyl residue.

All percentages are by weight unless otherwise stated.

In one aspect, the invention provides a composition, comprising
- at least one LC-PUFA,
- at least one probiotic, and
- a mixture of oligosaccharides, said mixture containing at least one N-acetylated oligosaccharide selected from the group comprising GalNAcα1,3Galβ1,4Glc (=3'GalNAc-I a c = N-acetyl-galactosaminyl-lactose) ,Galβ1,6GalNAcα1,3Galβ1,4Glc (= 6'Gal-3GalNAc-lac = galactosyl-N-acetyl-galactosaminyl-lactose), Galβ1,4GlcNAcβ1,3Galβ1,4Glc (lacto-N-neotetraose or LNnT) and Galβ1,3GlcNAcβ1,3Galβ1 (lacto-N-tetraose or LNT), at least one sialylated oligosaccharide selected from the group comprising NeuAcα2,3Galβ1,4Glc (= 3'-sialyllactose) and NeuAca2,6Galβ1,4Glc (= 6'-sialyllactose), and at least one neutral oligosaccharide selected from the group consisting of Galβ1,6Gal (=β1,6-digalactoside); Galß1,6Galβ1,4Glc (= 6'Gal-lac); Galß1,6Galβ1,6Glc; Galβ1,3Galβ1,3Glc; Galβ1,3Galβ1,4Glc (= 3'Gal-lac); Galβ1,6Galβ1,6Galβ1,4Glc (=6',6-diGal-lac); Galβ1,6Galβ1,3Galβ1,4Glc (=6',3-diGal-lac); Galβ1,3Galβ1,6Galβ1, 4 G I c (= 3 ' , 6-diGal-lac); Galβ1,3Galβ1,3Galβ1,4Glc (=3',3-diGal-lac); Galβ1,4Galβ1,4Glc (= 4' Gal-lac) and Galβ1,4Galβ1,4Galβ1,4Glc (=4',4-diGal-lac) ; and Fucα1,2Galβ1,4Glc (= 2' fucosyllactose or FL),
for use in brain growth and/or cognitive and/or psychomotor development.

In a second aspect, the invention relates to a composition comprising at least one long chain polyunsaturated fatty acid, at least one probiotic, and an oligosaccharide mixture which comprises:
- 0.25-20 wt%, preferably 0.3-10 wt%, more preferably 0.3-5 wt% and even more preferably around 0.5 wt%, with respect to the total weight of the oligosaccharide mixture, of at least one N-acetylated oligosaccharide,
- 0.5-30 wt%, preferably 0.75-15 wt%, more preferably 0.75-10 wt% and even more preferably around 1 wt%, with respect to the total weight of the oligosaccharide mixture, of at least one sialylated oligosaccharide, and
- 50-99.3 wt%, preferably 20-80 wt%, more preferably 10-50 wt% and even more preferably around 50 wt%, with respect to the total weight of the oligosaccharide mixture, of at least one neutral oligosaccharide,
for use in brain growth and/or cognitive and/or psychomotor development.

According to a preferred embodiment, the oligosaccharide mixture is present in an amount of 0.5-70%, more preferably 1-20%, even more preferably 2-5%, with respect to the total weight of the composition.

The oligosaccharide compounds are defined by their structures, where GalNAc is N-acetyl galactosamine, GlcNAc is N-acetyl glucosamine, Gal is galactose, NeuAc is N-acetyl neuraminic acid, Glc is glucose and Fuc is fucose.

The oligosaccharide mixture of the composition according to the invention can be the only source of oligosaccharide in the composition.

In a first embodiment, the neutral oligosaccharide is preferably chosen among FOS and GOS, preferably GOS such as the ones cited above.

In a second embodiment, independent or not from the first embodicment, the neutral oligosaccharide is preferably 2'-fucosyllactose (FL). In this case, FL is preferably included in the group of neutral oligosaccharides in the oligosaccharide mixture during its manufacturing.

The neutral oligosaccharide may be prepared as a mixture by purchasing and mixing the individual components. For example, synthesised galacto-oligosaccharides such as Galβ1,6Gal, Galß1,6Galβ1,4Glc, Galß1,6Galβ1,6Glc, Galβ1,3Galβ1,3Glc, Galβ1,3Galβ1,4Glc, Galβ1,6Galβ1,6Galβ1,4Glc, Galβ1,6Galβ1,3Galβ1,4Glc Galβ1,3Galβ1,6Galβ1,4Glc, Galβ1,3Galβ1,3Galβ1,4Glc, Galβ1,4Galβ1,4Glc and Galβ1,4Galβ1,4Galβ1,4Glc and mixtures thereof are commercially available under the trademarks Vivinal® from Friesland Campina, Netherlands, and Elix'or®. Other suppliers of oligosaccharides are Dextra Laboratories, Sigma-Aldrich Chemie GmbH and Kyowa Hakko Kogyo Co., Ltd. Alternatively, specific glycosyltransferases and/or glycosidases, such as galactosyltransferases, and/or fucosyltransferases and/or galactosidases and/or fucosidases may be used to produce galacto-oligosaccharides and/or fucosylated oligosaccharides.

The fucosyllactose is a fucosylated oligosaccharide (that is to say an oligosaccharide having a fucose residue). This fucosylated oligosaccharide may be isolated by chromatography or filtration technology from a natural source such as animal milks. Alternatively, it may be produced by biotechnological means using specific fucosyltransferases and/or fucosidase either through the use of enzyme-based fermentation technology (recombinant or natural enzymes) or microbial fermentation technology. In the latter case, microbes may either express their natural enzymes and substrates or may be engineered to produce respective substrates and enzymes. Single microbial cultures and/or mixed cultures may be used. Fucosylated oligosaccharide formation can be initiated by acceptor substrates starting from any degree of polymerization (DP), from DP = 1 onwards. Alternatively, fucosylated oligosaccharides may be produced by chemical synthesis from lactose and free fucose. Fucosylated oligosaccharides are also available for example from Kyowa, Hakko, Kogyo of Japan.

According to the invention, the sialylated oligosaccharide can be selected from the group comprising 3'-sialyllactose and 6'-sialyllactose. Preferably, the sialylated oligosaccharide comprises both 3'-sialyllactose and 6'-sialyllactose. In this embodiment, the ratio between 3'-sialyllactose and 6'-sialyllactose lies preferably in the range between 5:1 and 1:2.

The 3'- and 6'- forms of sialyllactose may be obtained by adding to the composition a natural source such as animal milk, or may be isolated by chromatographic or filtration technology from such natural source. Alternatively, they may be produced by biotechnological means using specific sialyltransferases or sialidases, neuraminidases, by an enzyme based fermentation technology (recombinant or natural enzymes), by chemical synthesis or by a microbial fermentation technology. In the latter case microbes may either express their natural enzymes and substrates or may be engineered to produce respective substrates and enzymes. Single microbial cultures or mixed cultures may be used. Sialyl-oligosaccharide formation can be initiated by acceptor substrates starting from any degree of polymerisation (DP), from DP=1 onwards. Alternatively, sialyllactoses may be produced by chemical synthesis from lactose and free N'-acetylneuraminic acid (sialic acid). Sialyllactoses are also commercially available for example from Kyowa Hakko Kogyo of Japan.

The N-acetylated oligosaccharides may be obtained by adding to the composition a natural source such as animal milk. Alternatively, they may be prepared by the action of glucosaminidase and/or galactosaminidase on N-acetyl-glucose and/or N-acetyl galactose. Equally, N-acetyl- galactosyl transferases and/or N-acetyl-glycosyl transferases may be used for this purpose. The N-acetylated oligosaccharides may also be produced through the use of fermentation technology using respective enzymes (recombinant or natural) and/or microbial fermentation. In the latter case the microbes may either express their natural enzymes and substrates or may be engineered to produce respective substrates and enzymes. Single microbial cultures or mixed cultures may be used. N-acetylated oligosaccharide formation can be initiated by acceptor substrates starting from any degree of polymerisation (DP), from DP=1 onwards. Another option is the chemical conversion of keto-hexoses (e.g. fructose) either free or bound to an oligosaccharide (e.g. lactulose) into N-acetylhexosamine or an N-acetylhexosamine containing oligosaccharide as described in Wrodnigg, T.M.; Stutz, A.E. (1999) Angew. Chem. Int. Ed. 38:827-828.

LNnT and LNT may be synthesised by enzymatic transfer of saccharide units from donor moieties to acceptor moieties using glycosylhydrolases and/or glycosyltransferases as described for example in US patent No. 5,288,637 and WO 96/10086. Alternatively, LNnT may be prepared by chemical conversion of Keto-hexoses (e.g. fructose) either free or bound to an oligosaccharide (e.g. lactulose) into N-acetylhexosamine or an N-acetylhexosamine-containing oligosaccharide as described in Wrodnigg, T.M.; Stutz, A.E. (1999) Angew. Chem. Int. Ed. 38:827-828. N-acetyl-lactosamine produced in this way may then be transferred to lactose as the acceptor moiety.

Preferably the N-acetylated oligosaccharide is selected from the group comprising lacto-N-neotetraose (or LNnT) and lacto-N-tetraose (or LNT). Preferably LNnT and/or LNT are included in the group of sialylated oligosaccharides in the oligosaccharide mixture during its manufacturing.

Probiotic bacterial strain present in the composition of the invention may be selected from any strain which satisfies the definition of a probiotic and has acceptable shelf-life for the composition in which it will be incorporated. For example, if the composition is incorporated into an infant formulae, said infant formulae is required to remain stable and effective for up to 12 months. The probiotic bacterial strain is preferably a *lactobacillus* or a *bifidobacterium.*

Examples of preferred *Bifidobacterium* species include *Bifidobacterium lactis, Bifidobacterium longum, Bifidobacterium breve and Bifidobacterium infantis,* Particularly preferred strains are *Bifidobacterium lactis CNCM I-3446* sold inter alia by the Christian Hansen company of Denmark under the trade mark Bb12, *Bifidobacterium longum* ATCC BAA-999 sold by Morinaga Milk Industry Co. Ltd. of Japan under the trade mark BB536, the strain of *Bifidobacterium breve* sold by Danisco under the trade mark Bb-03, the strain of *Bifidobacterium breve* sold by Morinaga under the trade mark M-16V, the strain of *Bifidobacterium infantis* sold by Procter & Gamble Co. under the trade mark Bifantis and the strain of *Bifidobacterium breve* sold by Institut Rosell (Lallemand) under the trade mark R0070 .

Examples of preferred *Lactobacillus* species are *Lactobacillus* rhamnosus, *Lactobacillus paracasei* and *Lactobacillus reuteri.* Particularly preferred strains are *Lactobacillus rhamnosus* ATCC 53103, *Lactobacillus rhamnosus* CGMCC 1.3724, *Lactobacillus reuteri* DSM 17938, and *Lactobacillus paracasei* CNCM I-2116. Even more preferably the probiotic is *Lactobacillus reuteri* DSM 17938.

*Lactobacillus reuteri* DSM 17938 is sold by BioGaia A.B under the trademark Reuteri.

According to the invention, the probiotic is chosen among probiotic bacterial strains, preferably the probiotic is a *lactobacillus* or a *bifidobacterium,* more preferably the probiotic is *Bifidobacterium lactis or Lactobacillus reuteri.*

The probiotic can be present in the composition in a wide range of percentages provided that the probiotic delivers the effect described. However, preferably, the probiotic is present in the composition in an amount equivalent to from 10e2 to 10e12 cfu (= colony forming unit) of probiotic bacterial strain, more preferably between 10e6 and 10e9 cfu, for each gram of the composition. This expression includes the possibilities that the bacteria are alive, inactivated or dead or even present as fragments such as DNA, cell wall materials, intracellular materials or bacteria metabolites. In other words, the quantity of bacteria which the composition contains is expressed in terms of colony forming ability of that quantity of bacteria if all the bacteria were live irrespective of whether they are, in fact, live, inactivated or dead, fragmented or a mixture of any or all of these states.

The composition contains at least one LC-PUFA, which is usually a n-3 or a n6 LC-PUFA. The n-3 LC-PUFA can be a C20 or a C22 n-3 fatty acid. The C20 or C22 n-3 LC-PUFA is preferably present in an amount of at least 0.1 wt% of all fatty acids in the composition. Preferably the n-3 LC-PUFA is docosahexanoic acid (DHA, C22:6, n-3). The n-6 LC-PUFA can be a C20 or a C22 n-6 fatty acid. The C20 or C22 n-6 LC-PUFA is preferably present in an amount of at least 0.1 wt% of all fatty acids in the composition. Preferably the n-6 LC-PUFA is arachidonic acid (ARA, C20:4, n-6). The source of LC-PUFA may be, for example, egg lipids, fungal oil, low EPA fish oil or algal oil. The LC-PUFA of the composition of the invention may be provided in small amounts of oils containing high quantities of preformed arachidonic acid and docosahexanoic acid such as fish oils or microbial oils.

The composition according to the invention is preferably a nutritional composition, more preferably a synthetic nutritional composition. In this case, it can be a preterm infant formula, a human milk fortifier, a starter infant formula, a follow-on formula, a baby food formula, an infant cereal formula, a growing-up milk, a medical food product for clinical nutrition, or a supplement, typically to be used during hospital stay and/or to be used after hospital discharge. A supplement can be for a preterm infant or a child or an adult. Said composition is preferably a product for preterm feeding such as a preterm infant formula, a human milk fortifier, or a preterm infant supplement. According to an embodiment, the composition is preferably a preterm infant formula, a human milk fortifier, or a supplement. The composition according to the invention can also be products for children or adults such as yogurt or medical food, as well as pets' food.

According to a particularly preferred embodiment, the composition according to the invention is for use in infants and young children who were born preterm or with LBW or experienced IUGR or who suffered from growth stunting due to disease and/or malnutrition, preferably preterm infants.

The composition according to the invention can be for use before and/or during and/or after a weaning period.

The invention includes also the use of a composition according to the invention, as a synthetic nutritional agent, for use in brain growth and/or cognitive and/or psychomotor development.
All the uses stated above are particularly intended for infants and young children, preferably infants, in case of humans. But these uses are also intended for young pets. The compositions and uses as per the present invention are particularly suited for infants and children, preferably infants, who were born preterm or with LBW or experienced IUGR or who suffered from growth delays because of disease and/or malnutrition, particularly during infancy.

Without wishing to be bound by theory, the inventors believe that the efficacy of the combination of oligosaccharide mixture in the composition described above in brain growth and/or cognitive and/or psychomotor development., may be the result of the synergistic combination of immunity modulator effects triggered by the probiotic bacterial strain and the LC-PUFA through their stimulation with the specific oligosaccharide mixture.

The oligosaccharide mixture, the LC-PUFA and the probiotic bacterial strain may be administered in the same composition or may be administered sequentially.

If the preterm and LBW infant group is to be addressed, the composition is preferably a nutritional composition, for example consumed in liquid form. It may be a nutritionally complete formula such as a (preterm) infant formula, a supplement, a human milk fortifier, a follow-on formula or a growing-up milk. Alternatively, for the group of young mammals, the composition may be a pet food.

The composition according to the invention can also contain a protein source. The type of protein is not believed to be critical to the present invention provided that the minimum requirements for essential amino acid content are met and satisfactory growth is ensured. Thus, protein sources based on whey, casein and mixtures thereof may be used as well as protein sources based on soy. As far as whey proteins are concerned, the protein source may be based on acid whey or sweet whey or mixtures thereof and may include alpha-lactalbumin and beta-lactoglobulin in any desired proportions. The proteins can be at last partially hydrolysed in order to enhancement of oral tolerance to allergens, especially food allergens. In that case the composition is a hypoallergenic composition.

The composition according to the present invention can also contain a carbohydrate source in addition to the oligosaccharide mixture. This is particularly preferable in the case where the composition of the invention is an infant formula. In this case, any carbohydrate source conventionally found in infant formulae such as lactose, saccharose, maltodextrin, starch and mixtures thereof may be used although the preferred source of carbohydrates is lactose. In any case, the oligosaccharide mixture is preferably the single source of prebiotic in the composition according to the invention.

The composition according to the present invention can also contain a source of lipids in addition to the LC-PUFA. This is particularly relevant if the nutritional composition of the invention is an infant formula. In this case, the lipid source may be any lipid or fat which is suitable for use in infant formulae. Preferred fat sources include palm oleic, high oleic sunflower oil and high oleic safflower oil. The essential fatty acids linoleic and α-linolenic acid may also be added. In the composition, the fat source (including the LC-PUFA such as ARA and/or DHA) preferably has a ratio of n-6 to n-3 fatty acids of about 1:2 to about 10:1, preferably about 3:1 to about 8:1.

The composition of the invention can also contain all vitamins and minerals understood to be essential in the daily diet and in nutritionally significant amounts. Minimum requirements have been established for certain vitamins and minerals. Examples of minerals, vitamins and other nutrients optionally present in the composition of the invention include vitamin A, vitamin B1, vitamin B2, vitamin B6, vitamin B12, vitamin E, vitamin K, vitamin C, vitamin D, folic acid, inositol, niacin, biotin, pantothenic acid, choline, calcium, phosphorous, iodine, iron, magnesium, copper, zinc, manganese, chlorine, potassium, sodium, selenium, chromium, molybdenum, taurine, and L-carnitine. Minerals are usually added in salt form. The presence and amounts of specific minerals and other vitamins will vary depending on the intended population. If necessary, the composition of the invention may contain emulsifiers and stabilisers such as soy, lecithin, citric acid esters of mono- and di-glycerides, and the like.

The composition of the invention may also contain other substances which may have a beneficial effect such as lactoferrin, nucleotides, nucleosides, gangliosides, polyamines, and the like.

The preparation of the composition according to the invention will now be described by way of example.

The formula may be prepared in any suitable manner. For example, it may be prepared by blending together a protein source, a carbohydrate source (different from the oligosaccharide mixture), and a fat source including the LC-PUFA in appropriate proportions. If used, the emulsifiers may be included at this point. The vitamins and minerals may be added at this point but are usually added later to avoid thermal degradation. Any lipophilic vitamins, emulsifiers and the like may be dissolved into the fat source prior to blending. Water, preferably water which has been subjected to reverse osmosis, may then be mixed in to form a liquid mixture. The temperature of the water is conveniently in the range between about 50°C and about 80°C to aid dispersal of the ingredients. Commercially available liquefiers may be used to form the liquid mixture. The oligosaccharide mixture will be added at this stage if the final product is to have a liquid form. If the final product is to be a powder, the oligosaccharides may likewise be added at this stage if desired. The liquid mixture is then homogenised, for example in two stages.

The liquid mixture may then be thermally treated to reduce bacterial loads, by rapidly heating the liquid mixture to a temperature in the range between about 80°C and about 150°C for a duration between about 5 seconds and about 5 minutes, for example. This may be carried out by means of steam injection, an autoclave or a heat exchanger, for example a plate heat exchanger.

Then, the liquid mixture may be cooled to between about 60°C and about 85°C for example by flash cooling. The liquid mixture may then be again homogenised, for example in two stages between about 10 MPa and about 30 MPa in the first stage and between about 2 MPa and about 10 MPa in the second stage. The homogenised mixture may then be further cooled to add any heat sensitive components, such as vitamins and minerals. The pH and solids content of the homogenised mixture are conveniently adjusted at this point.

The homogenised mixture is transferred to a suitable drying apparatus such as a spray dryer or freeze dryer and converted to powder. The powder should have a moisture content of less than about 5% by weight. The oligosaccharide mixture may be added at this stage by dry-mixing along with the probiotic bacterial strain(s), or by blending them in a syrup form of crystals, along with the probiotic bacterial strain(s), and spray-dry (or freeze -dry).

If a liquid composition is preferred, the homogenised mixture may be sterilised then aseptically filled into suitable containers or may be first filled into the containers and then retorted.

In another embodiment, the composition of the invention may be a supplement in an amount sufficient to achieve the desired effect in an individual. This form of administration is usually more suited to preterm or LBW or IUGR infants, older children and adults.

The amount of oligosaccharides, LC-PUFA and probiotic bacterial strain to be included in the supplement will be selected according to the manner in which the supplement is to be administered.

The supplement may be in the form of powder, tablets, capsules, pastilles or a liquid for example. The supplement may further contain protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co-compounds, dispersing agents, wetting agents, processing aids (solvents), flowing agents, taste masking agents, weighting agents, jellifying agents and gel forming agents. The supplement may also contain conventional pharmaceutical additives and adjuvants, excipients and diluents, including, but not limited to, water, gelatine of any origin, vegetable gums, lignin-sulfonate, talc, sugars, starch, gum arabic, vegetable oils, polyalkylene glycols, flavouring agents, preservatives, stabilizers, emulsifying agents, buffers, lubricants, colorants, wetting agents, fillers, and the like.

The supplement can be added in a product acceptable to the consumer (who is a human or an animal), such as an ingestible carrier or support, respectively. Examples of such carriers or supports are a pharmaceutical or a food or a pet food composition. Non-limiting examples for such compositions are milk, yogurt, curd, cheese, fermented milks, milk based fermented products, fermented cereal based products, milk based powders, human milk, preterm formula, infant formula, oral supplement, and tube feeding.

Further, the supplement may contain an organic or inorganic carrier material suitable for enteral or parenteral administration as well as vitamins, minerals trace elements and other micronutrients in accordance with the recommendations of Government bodies such as the USRDA.

The advantages, nature, and various additional features of the invention will appear more fully upon consideration of the illustrative experiment now to be described in detail in connection with accompanying drawings. In the drawings:

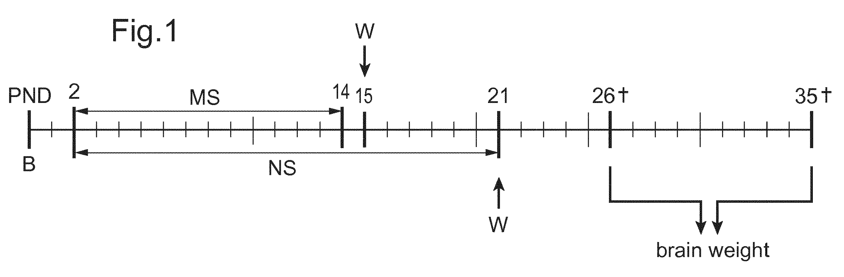

FIG. 1 is a diagram to illustrate the protocol of the experiments.
FIG. 2 is a bar graph plotting the results of the experiments, in terms of brain weight (g) at postnatal day (=PND) 35.
FIG. 3 is a bar graph plotting the results of the experiments, in terms of brain weight (g) at postnatal day (=PND) 26.

### Example

Experiments were carried out with respect to the effect of the supplementation of a oligosaccharide mixture which is a mixture of cow milk oligosaccharides (CMOS) enriched with galacto-oligosaccharides (demineralized, delactosed whey permeate or DDWP), LC-PUFA (arachidonic acid -ARA- and docosahexaenoic acid -DHA-), and *Bifidobacterium lactis* (BL), and optionally 2'-fucosyllactose (FL) or the combination of 2'-fucosyllactose (FL) and lacto N-neotetraose (LNNT), on pups.

### 1. Methodology

### Experimental protocol

The experiments were carried out in agreement with the Swiss Animal Protection Law (gravity degree 1) and were approved by the Office Vétérinaire Cantonal (Lausanne, Switzerland, authorization N° 2028). Reproductor male and virgin female Long-Evans Hooded rats were purchased from Janvier (France), arriving to the animal care facility two weeks before mating.
Pregnant females received food (Kliba 3437) and water *ad libitum,* were housed under constant temperature and humidity, and maintained on a 12:12 dark:light cycle. Housing conditions were kept for all the duration of the protocol. At postnatal day (=PND) 2 after birth (B), dams were removed from their maternity cages and the sex of the pups was determined. Standardized litters of 8 male pups were assigned for fostering, after randomizing by body weight. The dams and their pups were assigned to one of two rearing conditions: 1) maternal deprivation groups, exposed to a 180 min period of daily maternal separation on PND2 to PND14 **(MS),** or 2) unhandled controls **(NS).**

MS pups were weaned (W) at PND15. They were randomized by weight and nursing dam and distributed into groups of 16 animals which were fed till PND26 with either a control diet (modified AIN 93G, MS-Cont group) or a similar diet adapted to contain LC-PUFA, *Bifidobacterium lactis* CNCM I-3446 (BL) and oligosaccharides (DDWP). MS animals **(MS-Cont group)** were housed in groups of 8 pups up to PND21 to reduce the stress load of the premature weaning and then individually housed until the end of the experiment. Animals from the NS group were weaned onto the control diet **(NS-Cont group)** at PND21 and individually housed until the end of the experiment.

Animals were sacrificed (†) at PND26 or at PND26 by exhaustive bleeding under isoflurane anesthesia. Brain weight was recorded: the brain was collected after opening of the skull and weighted in a scale.

### 2. Treatment and diets

The following functional ingredients used for experimental gavage and diet composition comprised DDWP ingredient at 98.8 % dry matter, which composition is detailed in Table 1 below.

**Table 1. Composition of DDWP mixture**

| | % of Dry matter |
|---|---|
| Lactose | 33.4 |
| Total oligosaccharides | 25.51 |
| Glucose | 9.06 |
| Galactose | 8.13 |
| Protein | 4.03 |
| Ash | 11.43 |
| Unknown | 8.44 |

The DDWP is typically obtained according to the disclosures of WO2007/101675 or WO 2007/090894 and usually contains a mixture of about 30 wt% of GalNAcα1,3Galβ1,4Glc and Galβ1,6GalNAcα1,3Galβ1,4Glc ; 50 wt% of Galβ1,6Galβ1,4Glc and Galβ1,3Galβ1,4Glc ; 20 wt% of NeuAcα2,3Galβ1,4Glc and NeuAcα2,6Galβ1,4Glc.

Animals were fed from weaning till the end of the experiment with nutritionally adapted semisynthetic diets (modified AIN 93 G) which composition is shown in Tables 2, 3 and 4.

**TABLE 2: Diet recipes (per 100 g diet)**

| | **Control** | **PUFA-BL-DDWP** | **PUFA-BL-DDWP-FL** | **PUFA-BL-DDWP-FL-LNNT** |
|---|---|---|---|---|
| K-caseinate (g) | 20.00 | 20.00 | 20.00 | 20.00 |
| Corn starch (g) | 33.95 | 33.95 | 33.95 | 33.95 |
| Maltodextrin (g) | 20.00 | 13.69 | 13.85 | 14.05 |
| Sucrose (g) | 10.00 | 10.00 | 10.00 | 10.00 |
| Lactose (g) | 2.82 | - | 0.28 | 0.56 |
| Glucose (g) | 0.55 | - | 0.06 | 0.11 |
| Galactose (g) | 0.63 | - | 0.06 | 0.12 |
| DDWP (g)¹ | - | 9.94 | 8.95 | 7.95 |
| LNnT (g)² | - | - | - | 0.41 |
| FL(g)³ | - | - | 0.43 | 0.43 |
| Fat mix (g) (see below for composition) | 7.00 | 7.00 | 7.00 | 7.00 |
| Mineral mixture (AIN-93-G) (g) | 3.50 | 3.50 | 3.50 | 3.50 |
| Vitamine mixture (AIN-93-VX) (g) | 1.00 | 1.00 | 1.00 | 1.00 |
| L-Cysteine (g) | 0.30 | 0.30 | 0.30 | 0.30 |
| Cholinhydrogentartrate DAB 10 (g) | 0.25 | 0.25 | 0.25 | 0.25 |
| *B. lactis* powder (BL) (5.40E+10 cfu/g) (g)⁴ | | 0.37 | 0.37 | 0.37 |
| **Total (g)** | **100.00** | **100.00** | **100.00** | **100.00** |

| | | | | |
|---|---|---|---|---|
| *¹ DDWP* (demineralized, delactosed whey permeate); *²LNNT=Lacto-N-neoTetraose; ³FL=2-Fucosyl-Lactose; 4B. lactis=BL = B. lactis CNCM I-3446, spray-dried;* | | | | |

**Table 3: Fat mix (g/100 g fat mix)**

| **Control** | | **PUFA-BL-DDWP** | **PUFA-BL-DDWP-FL** | **PUFA-BL-DDWP-FL-LNNT** |
|---|---|---|---|---|
| Soybean oil | 21.80 | 21.90 | 21.90 | 21.90 |
| Cocoa butter | 37.34 | 27.41 | 27.41 | 27.41 |
| Corn oil | 40.86 | 40.10 | 40.10 | 40.10 |
| ARASCO (PUFA) | - | 5.15 | 5.15 | 5.15 |
| DHASCO (PUFA) | - | 5.44 | 5.44 | 5.44 |

**Table 4: Nutritional composition of diets**

| | **Control** | **PUFA-BL-DDWP** | **PUFA-BL-DDWP-FL** | **PUFA-BL-DDWP-FL-LNNT** |
|---|---|---|---|---|
| Digestible Kcal/100g (predicted)5 | 415 | 408 | 408 | 408 |
| Protein (g/100g, Nx6.25)⁶ | 17.87 | 18.36 | 18.20 | 18.11 |
| Fat (g/100g)⁷ | 7.11 | 7.09 | 7.03 | 7.17 |
| AA (%FA)⁸ | NA | NA | NA | NA |
| DHA(%FA)⁹ | NA | NA | NA | NA |
| *B lactis* (cfu/100 g diet)¹⁰ | ND | 1.24E+09 | 4.00E+09 | 1.89E+09 |

| | | | | |
|---|---|---|---|---|
| *⁵predicted from nutritional composition (1 g digestible carbohydrate=4 Kcal; 1 g oligosaccharide=2 Kcal; 1 g protein=4 Kcal; 1 g fat=9 Kcal); ⁶analyzed by Kjeldhal; ⁷ analyzed by Soxhlet; ⁸AA=arachidonic acid, ⁹DHA=docosahexaenoic acid; ¹⁰analyzed by standard culture method and PCR; NA= Not analyzed; ND=under detection limits (less than 1.00E*+*03)* | | | | |

The fatty acid profile of the four diets was balanced to provide similar ratio of n-6/n-3 and similar proportion of saturated, monounsaturated and polyunsaturated fatty acids. Thus, the fatty acid composition of the four diets was nearly the same in terms of fatty acid profile.

The animals were sacrificed on post natal day, PND, 26 or 35.

### 3. Brain weight

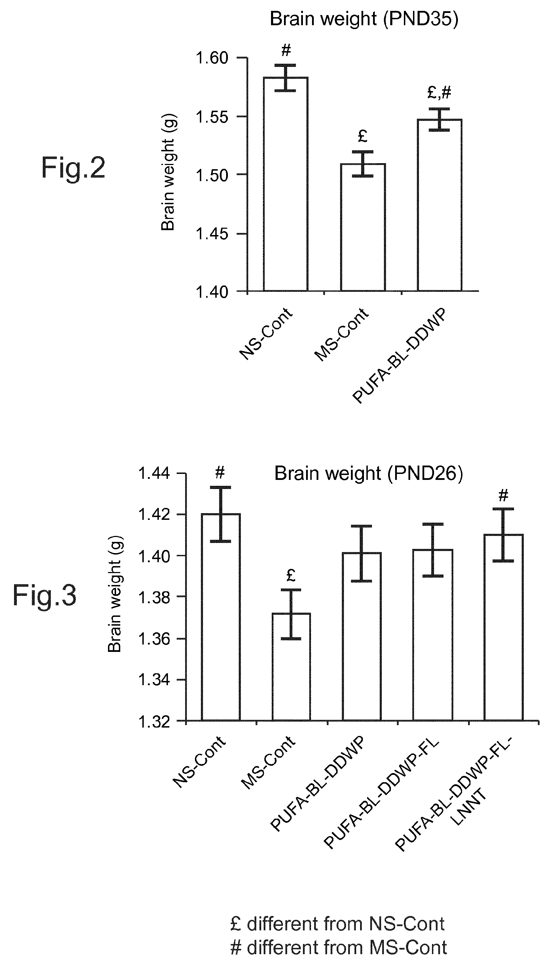

From the results on Figures 2 and 3, it appears that the first composition PUFA-BL-DDWP, according to the invention, show brain weight better than the brain weights obtained by the composition of control MS-CONT.

Furthermore, the third composition according to the invention PUFA-BL-DDWP-FL-LNNT showed a better brain weight than for the composition PUFA-BL-DDWP at PND 26. Actually, it appears that if DDWP-BL-PUFA was efficient at PND26, a longer supplementation at PND35 showed a more significant effect. DDWP-BL-PUFA-FL-LNNT showed already a significant effect at PND26.

All the compositions according to the invention proved to result in a heavier brain weight than the MS-CONT group. This is a real advantage of the compositions according to the invention.

## Claims

1. A composition comprising at least one long chain polyunsaturated fatty acid (LC-PUFA), at least one probiotic and a mixture of oligosaccharides, said mixture containing at least one N-acetylated oligosaccharide, at least one sialylated oligosaccharide and at least one neutral oligosaccharide, for use in brain growth, and/or cognitive and/or psychomotor development wherein, said composition is for use in infants and children, preferably infants, who were born preterm or with low-birth weight or experienced intra-uterine growth retardation or who suffered from growth stunting because of malnutrition and/or disease.

2. A composition according to the preceding claim, wherein the neutral oligosaccharide is chosen among fructooligosaccharides (FOS) and/or galactooligosaccharides (GOS), preferably GOS.

3. A composition according to any one of the preceding claims, wherein said oligosaccharide mixture contains at least one N-acetylated oligosaccharide selected from the group comprising GalNAcα1,3Galβ1,4Glc (=3'GalNAc-lac = N-acetyl-galactosaminyl-lactose), Galβ1,4GlcNAcβ1,3Galβ1,4Glc, Galβ1,3GlcNAcβ1,3Galβ1,4Glc and Galβ1,6GalNAcα1,3Galβ1,4Glc (= 6'Gal-3GalNAc-lac = galactosyl-N-acetyl-galactosaminyl-lactose), Galβ1.4GlcNAcβ1.3Galβ1.4Glc (lacto-N-neotetraose or LNnT) and Galβ1,3GlcNAcβ1,3Galβ1,4Glc (lacto-N-tetraose or LNT), at least one sialylated oligosaccharide selected from the group comprising NeuAcα2,3Galβ1,4Glc (= 3'-sialyllactose) and NeuAcα2,6Galβ1,4Glc (= 6'-sialyllactose), and at least one neutral oligosaccharide selected form the group consisting of Galβ1,6Gal (=β1,6-digalactoside); Galß1,6Galβ1,4Glc (= 6'Gal-lac); Galß1,6Galβ1,6Glc; Galβ1,3Galβ1,3Glc; Galβ1,3Galβ1,4Glc (= 3'Gal-lac); Galβ1,6Galβ1,6Galβ1,4Glc (=6',6-diGal-lac); Galβ1,6Galβ1,3Galβ1,4Glc (=6',3-diGal-lac); Galβ1,3Galβ1,6Galβ1,4Glc (=3',6-diGal-lac); Galβ1,3Galβ1,3Galβ1,4Glc (=3',3-diGal-lac); Galβ1,4Galβ1,4Glc (= 4' Gal-lac) and Galβ1,4Galβ1,4Galβ1,4Glc (=4',4-diGal-lac) ; and Fucα1,2Galβ1,4Glc (= 2' fucosyllactose or FL).

4. A composition according to any one of the preceding claims, wherein the oligosaccharide mixture comprises:
• 0.25-20 wt%, preferably 0.3-10 wt%, more preferably 0.3-5 wt% and even more preferably around 0.5 wt%, with respect to the total weight of the oligosaccharide mixture, of at least one N-acetylated oligosaccharide;
• 0.5-30 wt%, preferably 0.75-15 wt%, more preferably 0.75-10 wt% and even more preferably around 1 wt%, with respect to the total weight of the oligosaccharide mixture, of at least one sialylated oligosaccharide, and
• 50-99.3 wt%, preferably 20-80 wt%, more preferably 10-50 wt% and even more preferably around 50 wt%, with respect to the total weight of the oligosaccharide mixture, of at least one neutral oligosaccharide.

5. A composition according to any one of the preceding claims, wherein the oligosaccharide mixture comprises 0.5-70%, more preferably 1-20%, even more preferably 2-5%, with respect to the total weight of the composition.

6. A composition according to any one of the preceding claims, wherein the LC-PUFA is chosen among arachidonic acid (ARA) and docosahexanoic acid (DHA), preferably the LC-PUFA is a mixture of ARA and DHA.

7. A composition according to any one of the preceding claims, wherein the probiotic is chosen among probiotic bacterial strains, preferably the probiotic is a *lactobacillus* or a *bifidobacterium,* more preferably the probiotic is *Bifidobacterium lactis or Lactobacillus reuteri.*

8. A composition according to any one of the preceding claims, wherein the N-acetylated oligosaccharide is selected from the group comprising lacto-N-neotetraose (or LNnT) and lacto-N-tetraose (or LNT).

9. A composition according to any one of the preceding claims, wherein the sialylated oligosaccharide is selected from the group comprising 3'-sialyllactose and 6'-sialyllactose, and preferably the sialylated oligosaccharide comprises both 3'-sialyllactose and 6'-sialyllactose, the ratio between 3'-sialyllactose and 6'-sialyllactose lying preferably in the range between 5:1 and 1:2.

10. A composition according to any one of the preceding claims, wherein the neutral oligosaccharide is 2'-fucosyllactose (or FL).

11. A composition according to any one of the preceding claims, wherein said composition is a preterm infant formula, a human milk fortifier, a starter infant formula, a follow-on formula, a baby food formula, an infant cereal formula, a growing-up milk, a medical food product for clinical nutrition or a supplement and preferably, said composition is a preterm infant formula, a human milk fortifier, or a supplement.

## Patentansprüche

1. Zusammensetzung, die mindestens eine langkettige mehrfach ungesättigte Fettsäure (LC-PUFA), mindestens ein Probiotikum und eine Mischung aus Oligosacchariden umfasst, wobei die Mischung mindestens ein N-acetyliertes Oligosaccharid, mindestens ein sialyliertes Oligosaccharid und mindestens ein neutrales Oligosaccharid enthält, zur Verwendung für Gehirnwachstum und/oder kognitive und/oder psychomotorische Entwicklung, wobei die Zusammensetzung bei Säuglingen und Kindern verwendet wird, vorzugsweise bei Säuglingen, die zu früh geboren wurden oder ein geringes Geburtsgewicht aufwiesen oder intrauterine Wachstumsretardierung erlitten oder aufgrund von Mangelernährung und/oder Krankheit an Wachstumshemmung litten.

2. Zusammensetzung nach dem vorstehenden Anspruch, wobei das neutrale Oligosaccharid aus Fructooligosacchariden (FOS) und/oder Galactooligosacchariden (GOS), vorzugsweise GOS, ausgewählt wird.

3. Zusammensetzung nach einem der vorstehenden Ansprüche, wobei die
Oligosaccharidmischung mindestens ein N-acetyliertes Oligosaccharid ausgewählt aus der Gruppe umfassend GalNAcal ,3Galβ1,4Glc (=3'GalNAc-lac = N-Acetylgalactosaminyllactose), Galβ1,4GlcNAcβ1,3Galβ1,4Glc, Galβ1,3GlcNAcβ1,3Galβ1,4Glc und Galβ1,6GalNAcα1,3Galβ1,4Glc (= 6'Gal-3GalNAc-lac = Galactosyl-N-acetylgalactosaminyllactose), Galβ1,4GlcNAcβ1,3Galβ1,4Glc (Lacto-N-neotetraose oder LNnT) und Galβ1,3GlcNAcβ1,3Galβ1,4Glc (Lacto-N-tetraose oder LNT), mindestens ein sialyliertes Oligosaccharidausgewählt aus der Gruppe umfassend NeuAcα2,3Galβ1,4Glc (= 3'-Sialyllactose) und NeuAcα2,6Galβ1,4Glc (= 6'-Sialyllactose), und mindestens ein neutrales Oligosaccharid ausgewählt aus der Gruppe bestehend aus Galβ1,6Gal (=β1,6-Digalactosid); Galβ1,6Galβ1,4Glc (= 6'Gal-lac); Galβ1,6Galβ1,6Glc;Galβ1,3Galβ1,3Glc; Galβ1,3Galβ1,4Glc (= 3'Gal-lac); Galβ1,6Galβ1,6Galβ1,4Glc (=6',6-DiGal-lac); Galβ1,6Galβ1,3Galβ1,4Glc (=6',3-DiGal-lac) Galβ1,3Galβ1,6Galβ1,4Glc (=3',6-DiGal-lac); Galβ1,3Galβ1,3Galβ1,4Glc (=3',3-DiGal-lac); Galβ1,4Galβ1,4Glc (= 4' Gal-lac) und Galβ1,4Galβ1,4Galβ1,4Glc (=4',4-DiGal-lac); und Fucα1,2Galβ1,4Glc (= 2'-Fucosyllactose oder FL) enthält.

4. Zusammensetzung nach einem der vorstehenden Ansprüche, wobei die Oligosaccharidmischung Folgendes umfasst:
• 0,25-20 Gew.-%, vorzugsweise 0,3-10 Gew.-%, mehr bevorzugt 0,3-5 Gew.-% und noch mehr bevorzugt etwa 0,5 Gew.-%, mindestens eines N-acetylierten Oligosaccharids in Bezug auf das Gesamtgewicht der Oligosaccharidmischung;
• 0,5-30 Gew.-%, vorzugsweise 0,75-15 Gew.-%, mehr bevorzugt 0,75-10 Gew.-% und noch mehr bevorzugt etwa 1 Gew.-%, mindestens eines sialylierten Oligosaccharids in Bezug auf das Gesamtgewicht der Oligosaccharidmischung, und
• 50-99,3 Gew.-%, vorzugsweise 20-80 Gew.-%, mehr bevorzugt 10-50 Gew.-% und noch mehr bevorzugt etwa 50 Gew.-%, mindestens eines neutralen Oligosaccharids in Bezug auf das Gesamtgewicht der Oligosaccharidmischung.

5. Zusammensetzung nach einem der vorstehenden Ansprüche, wobei die Oligosaccharidmischung 0,5-70 %, mehr bevorzugt 1-20 % und noch mehr bevorzugt 2-5 -% der Mischung in Bezug auf das Gesamtgewicht der Zusammensetzung ausmacht.

6. Zusammensetzung nach einem der vorstehenden Ansprüche, wobei die LC-PUFA ausgewählt ist aus Arachidonsäure (ARA) und Docosahexaensäure (DHA) und es sich vorzugsweise bei der LC-PUFA um eine Mischung aus ARA und DHA handelt.

7. Zusammensetzung nach einem der vorstehenden Ansprüche, wobei das Probiotikum aus Bakterienstämmen ausgewählt ist und es sich vorzugsweise bei dem Probiotikum um einen *Lactobacillus* oder ein *Bifidobacterium* handelt und es sich mehr bevorzugt bei dem Probiotikum um *Bifidobacterium lactis* oder *Lactobacillus reuteri* handelt.

8. Zusammensetzung nach einem der vorstehenden Ansprüche, wobei das N-acetylierte Oligosaccharid ausgewählt ist aus der Gruppe umfassend Lacto-N-neotetraose (oder LNnT) und Lacto-N-tetraose (oder LNT).

9. Zusammensetzung nach einem der vorstehenden Ansprüche, wobei das sialylierte Oligosaccharid ausgewählt ist aus der Gruppe umfassend 3'-Sialyllactose und 6'-Sialyllactose, und vorzugsweise umfasst das sialylierte Oligosaccharid sowohl 3'-Sialyllactose als auch 6'-Sialyllactose, wobei das Verhältnis von 3'-Sialyllactose zu 6'-Sialyllactose vorzugsweise im Bereich von 5:1 bis 1:2 liegt.

10. Zusammensetzung nach einem der vorstehenden Ansprüche, wobei es sich bei dem neutralen Oligosaccharid um 2'-Fucosyllactose (oder FL) handelt.

11. Zusammensetzung nach einem der vorstehenden Ansprüche, wobei es sich bei der Zusammensetzung um eine Säuglingsnahrung für Frühgeborene, ein Anreicherungsmittel für Muttermilch, eine Säuglingsanfangsnahrung, eine Säuglingsfolgenahrung, eine Babynahrung, eine Säuglingsgetreidenahrung, eine Wachstumsmilch, ein medizinisches Nahrungsmittelprodukt für die klinische Ernährung oder ein Ergänzungsmittel handelt und wobei es sich vorzugsweise bei der Zusammensetzung um eine Säuglingsnahrung für Frühgeborene, ein Anreicherungsmittel für Muttermilch oder ein Ergänzungsmittel handelt.

## Revendications

1. Composition comprenant au moins un acide gras polyinsaturé à longue chaîne (LC-PUFA), au moins un probiotique et un mélange d'oligosaccharides, ledit mélange contenant au moins un oligosaccharide N-acétylé, au moins un oligosaccharide sialylé et au moins un oligosaccharide neutre, destinée à être utilisée dans la croissance du cerveau, et/ou le développement cognitif et/ou psychomoteur, ladite composition étant destinée à être utilisée chez les nourrissons et les enfants, de préférence les enfants qui sont nés prématurés ou de faible poids à la naissance ou qui ont fait l'objet d'un retard de croissance intra-utérine ou qui ont souffert d'un retard de croissance en raison d'une malnutrition et/ou d'une maladie.

2. Composition selon la revendication précédente, dans laquelle l'oligosaccharide neutre est choisi parmi des fructo-oligosaccharides (FOS) et/ou des galacto-oligosaccharides (GOS), de préférence les GOS.

3. Composition selon l'une quelconque des revendications précédentes, dans laquelle ledit
mélange d'oligosaccharides contient au moins un oligosaccharide N-acétylé choisi dans le groupe comprenant GalNAcα1,3Gal1,4Glc (=3'GalNAc-lac = N-acétyl-galactosaminyl-lactose), Galβ1,4GlcNAcβ1,3Galβ1,4Glc, Galβ1,3GlcNAcβ1,3Galβ1,4Glc et Galβ1,6GalNAcα1,3Galβ1,4Glc (= 6'Gal-3GaINAc-lac = galactosyl-N-acétyl-galactosaminyl-lactose), Galβ1,4GlcNAcβ1,3Galβ1,4Glc (lacto-N-néotétrose ou LNnT) et Galβ1,3GlcNAcβ1,3Galβ1,4Glc (lacto-N-tétraose ou LNT), au moins un oligosaccharide sialylé choisi dans le groupe comprenant NeuAcα2,3Galβ1,4Glc (= 3'-sialyllactose) et NeuAcα2,6Galβ1,4Glc (= 6'-sialyllactose), et au moins un oligosaccharide neutre choisi dans le groupe comprenant Galβ1,6Gal (=β1,6-digalactoside) ; Galβ1,6Galβ1,4Glc (= 6'Gal-lac) ; Galβ1,6Galβ1,6Glc;Galβ1,3Galβ1,3Glc ; Galβ1,3Galβ1,4Glc (= 3'Gal-lac) ; Galβ1,6Galβ1,6Galβ1,4Glc (=6',6-diGal-lac) ; Galβ1,6Galβ1,3Galβ1,4Glc (=6',3-diGal-lac) Galβ1,3Galβ1,6Galβ1,4Glc (=3',6-diGal-lac) ; Galβ1,3Galβ1,3Galβ1,4Glc (=3',3-diGal-lac) ; Galβ1,4Galβ1,4Glc (= 4' Gal-lac) et Galβ1,4Galβ1,4Galβ1,4Glc (=4',4-diGal-lac) ; et Fucα1,2Galβ1,4Glc (= 2'fucosyllactose ou FL).

4. Composition selon l'une quelconque des revendications précédentes, dans laquelle le mélange d'oligosaccharides comprend :
• de 0,25 à 20 % en poids, de préférence de 0,3 à 10 % en poids, plus préférablement de 0,3 à 5 % en poids, et encore plus préférablement environ 0,5 % en poids, par rapport au poids total du mélange d'oligosaccharides, d'au moins un oligosaccharide N-acétylé ;
• de 0,5 à 30 % en poids, de préférence de 0,75 à 15 % en poids, plus préférablement de 0,75 à 10 % en poids, et encore plus préférablement environ 1 % en poids, par rapport au poids total du mélange d'oligosaccharides, d'au moins un oligosaccharide sialylé, et
• de 50 à 99,3 % en poids, de préférence de 20 à 80 % en poids, plus préférablement de 10 à 50 % en poids, et encore plus préférablement environ 50 % en poids, par rapport au poids total du mélange d'oligosaccharides, d'au moins un oligosaccharide neutre.

5. Composition selon l'une quelconque des revendications précédentes, dans laquelle le mélange d'oligosaccharides constitue de 0,5 à 70 %, plus préférablement de 1 à 20 %, encore plus préférablement de 2 à 5 %, par rapport au poids total de la composition.

6. Composition selon l'une quelconque des revendications précédentes, dans laquelle le LC- PUFA est choisi parmi l'acide arachidonique (ARA) et l'acide docosahexanoïque (DHA), de préférence le LC-PUFA est un mélange d'ARA et de DHA.

7. Composition selon l'une quelconque des revendications précédentes, dans laquelle le probiotique est choisi parmi les souches bactériennes probiotiques, de préférence le probiotique est un *lactobacillus* ou un *bifidobacterium,* plus préférablement, le probiotique est le *Bifidobacterium lactis* ou le *Lactobacillus reuteri.*

8. Composition selon l'une quelconque des revendications précédentes, dans laquelle l'oligosaccharide N-acétylé est choisi dans le groupe comprenant le lacto-N-néotétraose (ou LNnT) et le lacto-N-tétraose (ou LNT).

9. Composition selon l'une quelconque des revendications précédentes, dans laquelle l'oligosaccharide sialylé est choisi dans le groupe comprenant le 3'-sialyllactose et le 6'-sialyllactose, et de préférence l'oligosaccharidique sialylé comprend à la fois le 3'-sialyllactose et le 6'-sialyllactose, le rapport entre le 3'-sialyllactose et le 6'-sialyllactose se situant de préférence dans la plage comprise entre 5:1 et 1:2.

10. Composition selon l'une quelconque des revendications précédentes, dans laquelle l'oligosaccharide neutre est le 2'-fucosyllactose (ou FL).

11. Composition selon l'une quelconque des revendications précédentes, où ladite composition est une préparation pour enfants prématurés, un fortifiant de lait humain, une préparation pour nourrissons à base de ferments lactiques, une préparation de suite, une préparation d'aliments pour bébé, une préparation de céréales pour nourrissons, un lait de croissance, un produit alimentaire médical pour la nutrition clinique ou un complément et, de préférence, ladite composition est une préparation pour enfants prématurés, un fortifiant de lait humain, ou un complément.
